Europäisches Patentamt

European Patent Office

Office européen des brevets

(11) Veröffentlichungsnummer: **0 291 696**
**A2**

## (12) EUROPÄISCHE PATENTANMELDUNG

(21) Anmeldenummer: 88106036.2

(51) Int. Cl.⁴: **C12N 5/00**

(22) Anmeldetag: 15.04.88

(30) Priorität: 20.05.87 DE 3716907

(43) Veröffentlichungstag der Anmeldung:
23.11.88 Patentblatt 88/47

(84) Benannte Vertragsstaaten:
**DE FR GB IT**

(71) Anmelder: **Battelle-Institut e.V.**
**Am Römerhof 35 Postfach 900 160**
**D-6000 Frankfurt/Main 90(DE)**

(72) Erfinder: **Dreher, Ralf Michael, Dr.**
**Oberer Dorfgraben 61**
**D-6500 Mainz-Laubenheim(DE)**
Erfinder: **Knoell, Hans-Emil, Dr.**
**Hubestrasse 27**
**D-6940 Weinheim(DE)**

(74) Vertreter: **Sartorius, Peter, Dipl.-Ing.**
**Battelle-Institut e.V. Abteilung Patente Am**
**Römerhof 35**
**D-6000 Frankfurt am Main 90(DE)**

(54) Verfahren zur Vermehrung von Epithelialzellen.

(57) Verfahren zur Steigerung des Wachstums von Epithelialzellen in einer Forskolin enthaltenen Kultur.

EP 0 291 696 A2

## Verfahren zur Vermehrung von Epithelialzellen

Die Erfindung betrifft ein Verfahren zur Vermehrung von menschlichen oder tierischen Epitheliazellen in einer Kultur, in der sie mit einem das Wachstum der Epithelialzellen beschleunigenden Mittel in Berührung gebracht werden.

Bei der Behandlung großflächiger Brandwunden konnte mit den bisherigen Behandlungsmethoden, wie Kadaver-Allotransplantate, Xenotransplantate von Schweinehaut und künstlicher Haut, keine wesentlichen Fortschritte erzielt werden. Die Anwendung dieser Methode beinhaltet zwar eine Lebensverlängerung, eine permanente Heilung ist aber aufgrund von immunologischen Abstoßungsreaktionen bis heute nicht möglich.

Eine Lösung dieses Problems scheint durch eine Methode von Prof. Howard Green aus Boston, (H. Green et al, Proc. Natl. Acad. Sci. USA 76 (1979),5665), möglich zu sein. Hierzu werden menschliche epidermale Zellen, die mittels einer Hautbiopsie (ca. 2 cm$^2$) entnommen werden, in Zellkulturen vermehrt. Nach mehreren Wochen ist die Expansion der Epithelialzellen soweit fortgeschritten, daß sie mit Hilfe eines Enzyms als Zellverband entnommen und als autologe Zellkulturhaut zur Transplation der Brandverletzungen benutzt werden kann.

Bie der Kultivierung primärer menschlicher Epithelialzellen ergeben sich aber zwei Probleme:

1. Bei der Gewinnung der Epithelialzellen aus Hautbiopsien gewinnt man auch andere Zelltpyen, hauptsächlich Fibroblasten. Diese Fibroblasten wachsen schneller und besser als die Epithelialzellen und überwuchern diese in kurzer Zeit. Dieses Problem kann dadurch gelöst werden, indem zusammen mit den Epithelialzellen bestrahlte Fibroblastenzellen, die aufgrund des Strahlungsschadens sich nicht mehr teilen können, angesät werden. Diese auch als Feederlayer bezeichneten bestrahlten Fibroblasten verhindern das Wachstum von Fibroblasten, die bei der Gewinnung der Epithelialzellen ebenfalls anfallen.

2. Primäre humane Epithelialzellen zeigen bei der in vitro Kultivierung ein sehr geringes Wachstum. In einigen Veröffentlichungen von M. Green und J. Rheinwald (1. J.G. Rheinwald und H. Green, Cell 6 - (1975), 317; 2. J.G. Rheinwald und H. Green, Cell 6 (1975), 331; 3. H. Green, Cell 15 (1978), 801) ist beschrieben, daß man durch Erhöhung des Intrazellulären cyclo-AMP-Spiegels ein günstigeres Wachstum bekommt.

Diese Forschungsgruppe konnte vier Substanzen (1.) Cholera Toxin, 2.) Dibutyryl-cyclo-AMP, 3.) Methyl-isobutylxanthin, 4.) Isoproterenol) identifizieren, die das Wachstum der Epithelialzellen günstig beeinflussen. Allen gemeinsam ist eine Induktion und Steigerung des cyclo-AMP-Spiegels. Diese vier Substanzen werden im Zusammenhang mit der Kultivierung der Epithelialzellen im US-Patent 4,456,678 beschrieben. Von diesem Stand der Technik geht die Erfindung aus.

Die Wachstumsgeschwindigkeit der Epithelialzellen ist bei einer Verwendung der Zellen als Transplantat für Schwerverletzte essentiell. Da nach ca. drei Wochen die Sepsisgefahr bei diesen Patienten wächst, muß innerhalb dieser drei Wochen ca. 1 - 2 m$^2$ Zellkulturhaut produziert werden.

Es konnte eine Substanz identifiziert werden, die das Waschstum humaner epidermaler Zellen im Vergleich zu Cholera Toxin, der Sustanz mit den bisher günstigsten Resultaten, erheblich steigerte. Bei der Substanz handelt es sich um Forskolin, ein Diterpen, das aus der Pflanze Coleus forsikolii isoliert wird. Forskolin bindet direkt an das Enzym Adenylat Cyklase, das für die Synthese von cyclo-AMP verantwortlich ist, und stimuliert dieses permanent. In diesem Punkt unterscheidet sich Forskolin sehr stark von Substanzen wie Cholera Toxin oder Isoproterenol, die nicht direkt mit der Adenylat Cyklase reagieren, sondern indirekt, indem sogenannte G-Proteine aktiviert werden, die wiederum die Adenylat Cyklase aktivieren.

Letztlich führen sowohl Forskolin als auch Cholera Toxin zu einem höheren cyclo-AMP-Level in der Zelle, wobei Forskolin aufgrund der direkten Bindung und Aktivierung der Adenylat Cyklase zu einer deutlich höheren Aktivierung der cyclo-AMP-Produktion führt als dies durch Cholera Toxin der Fall ist, das über eine ADP-Ribosylierung eines G-Proteins indirekt die Adenylat Cyklase stimuliert.

Alle hier beschriebenen Experimente mit Forskolin werden verglichen mit Cholera Toxin in einer Konzentration von $10^{-9}$ M. Die Wirkung von Forskolin bei der Kultivierung humaner Epithelialzellen ist stark dosisabhängig. Bei einer Konzentration von $10^{-5}$ M bzw. $5 \times 10^{-6}$ M Forskolin is das beste Waschstum zu beobachten. Doch auch bei $10^{-6}$ M ist das beobachtete Zellwachstum noch größer als mit Cholera Toxin. Der Effekt bei $10^{-7}$ M Forskolin entspricht in etwa der Cholera Toxin-Wirkung.

Die Anmeldung kann durch folgende Beispiele näher erläutert werden.

Beispiel 1

## Effekt von Forskolin

Kerationozyten wurden durch Trypsinierung von Haut nach der Methode von M. Green (Green, M. et al, PNAS 76 (1979) 5665) gewonnen und in einer Zelldichte von $2,5 \times 10^4$ Zellen/cm$^2$ ausgesät. Die Hautproben in den Beispielen 1, 2 und 3 stammten von Patienten die zwischen 30 und 40 Jahre alt waren. Zusätzlich wurden die Kulturen mit $2 \times 10^4$ Zellen/cm$^2$ letal bestrahlter 3T3-Fibroblasten inokuliert, beschrieben im US-Patent 4,016,036.

Als Medium diente eine Mischung aus DMEM und HAM's F 12 (3:1) mit folgenden Zusätzen:

Hydrocortison 0,4 ug/ml

Insulin 5 ug/ml

Triiodo-Thyronin $2 \times 10^{-9}$ M

Transferrin 5 ug/ml

Adenin $8 \times 10^{-4}$ M

Gentamycin, Penicillin, Streptomycin je 50 ug/ml

Fötales Kälberserum 10 %

EGF (nach 2 Tagen) 10 ng/ml

Um den Effekt von Forskolin im Vergleich mit Cholera Toxin zu untersuchen, wurden Parallelansätze mit unterschiedlichen Konzentrationen Forskolin ($10^{-5}$ - $10^{-8}$ M) und mit Cholera Toxin $10^{-9}$ M versetzt. Nach 10 Tagen wurden die Proben fixiert und die Keratinozytenkolonien mit Rhodamin B gefärbt. Es konnte eine deutliche Steigerung der Größe der Keratinozytenkolonien bei einer Forskolinkonzentration von $10^{-5}$ M - $10^{-6}$ M festgestellt werden. Eine Forskolinkonzentration von $10^{-7}$ M zeigte noch die gleichen Resultate wie bei Zugabe von Cholera Toxin, $10^{-8}$ M Forskolin war deutlich schlechter als Cholera Toxin.

## Beispiel 2

Bei einem ähnlichen Experiment wie unter Beispiel 1 beschrieben, wurden die Keratinozytenkulturen mit unterschiedlichen Konzentrationen Forskolin und mit Cholera Toxin bis zum Erreichen der Konfluenz gezüchtet. Zusätzlich wurden nach 12 Tagen die Zellen abgelöst und gezählt.

| Probe | Zellzahl nach 12 Tagen in einer 25cm$^2$ Kulturschale | Anzahl der Tage bis zur Konfluenz |
|---|---|---|
| Cholera Toxin $10^{-9}$ M | $6,9 \times 10^5$ | 24 |
| Forskolin $10^{-8}$ M | $2,5 \times 10^5$ | keine Konfluenz |
| Forskolin $10^{-7}$ M | $8 \times 10^5$ | 24 |
| Forskolin $10^{-6}$ M | $12 \times 10^5$ | 18 |
| Forskolin $5 \times 10^{-6}$ M | $23 \times 10^5$ | 14 |
| Forskolin $10^{-5}$ M | $25 \times 10^5$ | 14 |
| Forskolin $5 \times 10^{-5}$ M | $13 \times 10^5$ | 18 |

Diese Daten zeigen deutlich, daß bei einer Forskolinkonzentration von $5 \times 10^{-6}$ bzw. $10^{-5}$ im Medium sich die Zeit bis zum Erreichen einer konfluenten Keratinozytenkultur im Vergleich zu Cholera Toxin um bis zum 40 % resuzieren läßt.

Beispiel 3

In diesem Experiment, das wie unter Beispiel 1 beschrieben durchgeführt wurde, wurden verschiedene Forskolinkonzentrationen unter Verwendung bzw. in Vergleich von 10 % fötalem Kälberserum (FCS) und einem synthetischen Serum der Firma Biomedica Wien (Nu-Serum Nr. 1) untersucht.

| Probe | Zellzahl nach 12 Tagen in einer 25cm$^2$ Kultur-schale | Anzahl der Tage bis zur Konfluenz |
|---|---|---|
| FCS, Forskolin $10^{-6}$M | $14 \times 10^5$ | 20 |
| FCS, Forskolin $5\times10^{-6}$M | $24 \times 10^5$ | 15 |
| FCS, Forskolin $10^{-5}$M | $32 \times 10^5$ | 14 |
| FCS, Forskolin $5\times10^{-5}$M | $16 \times 10^5$ | 18 |
| FCS, Cholera Toxin $10^{-9}$M | $7 \times 10^5$ | 23 |
| Nu-Serum, Forskolin $10^{-6}$M | $14 \times 10^5$ | 20 |
| Nu-Serum, Forskolin $5\times10^{-6}$M | $25 \times 10^5$ | 16 |
| Nu-Serum, Forskolin $10^{-5}$M | $27 \times 10^5$ | 15 |
| Nu-Serum, Forskolin $5\times10^{-5}$M | $13 \times 10^5$ | 18 |
| Nu-Serum, Cholera Toxin $10^{-9}$ | $8 \times 10^5$ | 23 |

Auch dieses Experiment zeigte deutlich, daß Forskolin unabhängig von der Wahl des Serums in einem Konzentrationsbereich von ca. $5 \times 10^{-6}$ bis $10^{-5}$M das Wachstum der Keratinozyten erheblich beschleunigt und somit die Zeit bis zum Erreichen von konfluenten Kulturen und damit auch bis zum Erreichen von Zellkulturhaut-Transplantation verkürzt.

**Ansprüche**

1. Verfahren zu Vermehrung von menschlichen oder tierischen Epithelialzellen in einer Kultur, in der sie mit einem das Wachstum der Epithelialzellen beschleunigenden Mittel in Berührung gebracht werden, **dadurch gekennzeichnet,** daß die Epithelialzellen mit Forskolin in Berührung gebracht werden.

2. Verfahren nach Anspruch 1, **dadurch gekennzeichnet,** daß Forskolin in einer Konzentration von etwa $10^{-5}$M bis $10^{-6}$M, vorzugweise bis etwa $5 \times 10^{-6}$M, verwendet wird.